(19) Europäisches Patentamt
European Patent Office
Office européen des brevets

(11) Veröffentlichungsnummer: **0 245 774 B1**

(12) # EUROPÄISCHE PATENTSCHRIFT

(45) Veröffentlichungstag der Patentschrift: **05.12.90**

(51) Int. Cl.[5]: **C07C 213/00,** C07C 215/68, C07H 5/04, C07C 215/10

(21) Anmeldenummer: **87106599.1**

(22) Anmeldetag: **07.05.87**

(54) **Verbessertes Verfahren zur Herstellung von Ribitylxylidin.**

(30) Priorität: **10.05.86 DE 3615834**

(43) Veröffentlichungstag der Anmeldung: **19.11.87 Patentblatt 87/47**

(45) Bekanntmachung des Hinweises auf die Patenterteilung: **05.12.90 Patentblatt 90/49**

(84) Benannte Vertragsstaaten: **CH DE FR GB LI NL**

(56) Entgegenhaltungen:
**EP-A- 0 046 495**
**US-A- 2 429 244**

(73) Patentinhaber: **BASF Aktiengesellschaft, Carl-Bosch-Strasse 38, D-6700 Ludwigshafen(DE)**

(72) Erfinder: **Ernst, Hansgeorg, Dr., Bruesseler Ring 38, D-6700 Ludwigshafen(DE)**
Erfinder: **Leininger, Hartmut, Dr., Snerlevej 9, DK-8500 Greenaa(DK)**
Erfinder: **Paust, Joachim, Dr., Ringstrasse 3, D-6708 Neuhofen(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Herstellung von N-(3,4-Dimethyl-phenyl)-D-1-ribitylamin (Ribitylxylidin; I) durch Umsetzen von (D)-Ribose mit 3,4-Dimethyl-anilin bei erhöhter Temperatur und unter hydrierenden Bedingungen. I ist ein wichtiges Zwischenprodukt bei der industriellen Herstellung von Riboflavin (Vitamin $B_2$).

I erhält man durch reduktive Aminierung von D-Ribose (II) mit 3,4-Dimethyl-anilin (III) oder 3,4-Dimethyl-nitrobenzol (IV) (vgl. Ullmanns Encyklopädie der technischen Chemie, 4. Aufl., Bd. 23 (1983), S. 666/67).

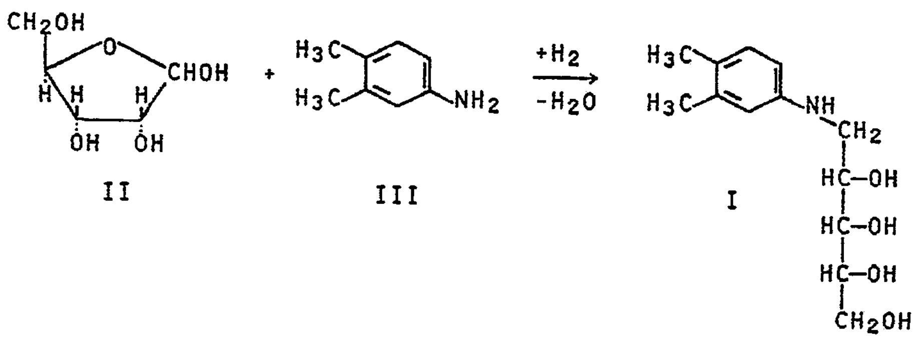


II III I

Im technischen Maßstab wird diese reduzierende Kondensation als katalytische Hydrierung ausgeführt.

Die direkte Umsetzung von II mit III in Gegenwart von $H_2$ und Raney-Nickel zu I ohne Isolierung des als Zwischenprodukt auftretenden Ribosids V

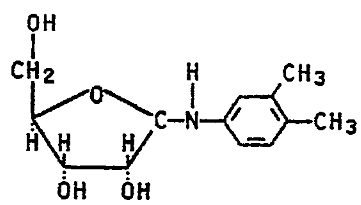

(V)

wird in US 2 477 560 beschrieben. Nachteilig an diesem Verfahren ist, daß Wasserstoffdrucke von etwa 25 bis 50 bar notwendig sind, um gute Ausbeuten zu erzielen.

Aus US 2 429 244 ist ein Verfahren zur Herstellung von I bekannt, bei dem nicht nur kristalline Ribose, sondern auch Lösungen von Rohribose eingesetzt werden können. Gemäß diesem Verfahren wird die D-Riboselösung mit mindestens 1 Mol Borsäure und mit 1 Mol 3,4-Dimethyl-anilin pro Mol Ribose versetzt und das dabei erhaltene kristalline Borsäure enthaltende Zwischenprodukt nach Abtrennen und Waschen in alkanolischer Lösung katalytisch hydriert. Auch bei diesem Verfahren sind relativ hohe Wasserstoffdrucke notwendig. Die Ribitylxylidin-Ausbeuten betragen ca. 75 bis 78 % der Theorie.

Auch gemäß EP 46 495 wird I dadurch erhalten, daß man Roh-Ribose mit 3,4-Dimethyl-anilin und äquimolaren Mengen Borsäure in wäßriger oder wäßrig-organischer Lösung umsetzt, den hierbei gebildeten kristallinen Borsäureester isoliert und anschließend diesen Borsäureester in wäßriger oder wäßrig-organischer Lösung bei 20 bis 70°C unter einem Wasserstoffdruck von 10 bis 100 bar, vorzugsweise bei 30 bar, in Gegenwart eines Hydrierkatalysators hydriert. Die Ausbeuten betragen hierbei etwa 85,5 %, bezogen auf Ribose. Nachteilig auch hierbei sind die hohen Wasserstoffdrucke sowie der Einsatz von äquimolaren Mengen an Borsäure und die dadurch bedingten Aufarbeitungsprobleme.

Weiterhin ist aus DE-OS 3 004 304 und DE-OS 2 923 268 die I-Herstellung auf folgendem Wege bekannt. Durch Epimerisierung von D-Arabinose mit Molybdän (VI)-Verbindungen und anschließend Grobabtrennung von unumgesetzter Arabinose durch Kristallisation wird eine Rohribose erhalten, die bei einem Wasserstoffdruck von 3 bar unter Verwendung von Pd/C anstelle von Raney-Nickel als Hydrierkatalysator mit 3,4-Dimethyl-anilin oder mit 3,4-Dimethyl-nitrobenzol, das in situ zu 3,4-Dimethylanilin reduziert wird, zu I umgesetzt werden kann. Die Ausbeuten an I gemäß diesem Verfahren betragen jedoch nur etwa 78,5 %.

Gemäß DE-OS 34 37 571 wird bei der oben beschriebenen Epimerisierung von D-Arabinose mit Molybdän(VI)-Verbindungen ein weit höherer Epimerisierungsgrad erzielt, wenn man die Epimerisierung in Gegenwart einer Borsäureverbindung durchführt. Die Borsäureverbindung wird dabei in einer Menge verwendet, die mindestens der 0,5-fachen oder vorzugsweise der 1,5-fachen Molarmenge an D-Arabinose

entspricht und wird nach der Epimerisierung im allgemeinen durch Ausfällen mit Methanol, elektrische Dialyse und/oder durch Behandlung der Lösung mit einem schwach basischen Ionenaustauscher-Harz entfernt. Anschließend wird bei diesem Verfahren die Ribose bei hohen Wasserstoffdrucken (40 bis 50 bar) mit 3,4-Dimethyl-anilin zu I umgesetzt.

Es war daher die Aufgabe der Erfindung die Herstellung von I durch hydrierende Kondensation von II mit III oder IV so zu verbessern, daß man I trotz Anwendung von nur niederen Wasserstoffdrücken auf möglichst einfache Weise in sehr guten Ausbeuten erhält.

Es wurde nun überraschenderweise gefunden, daß man beim Umsetzen von II mit III oder IV in wäßrig oder organisch-wäßriger Lösung in Gegenwart von Wasserstoff und einem Hydrierkatalysator bei Verwendung von Raney-Nickel als Katalysator auch bei Anwendung nur niederer Wasserstoffdrücke I in sehr guten Ausbeuten von 87 bis über 90 % der Theorie, bezogen auf Ribose, erhält, wenn man die Umsetzung in Gegenwart von katalytischen Mengen einer geeigneten Borsäureverbindung durchführt.

Gegenstand der Erfindung ist dementsprechend ein Verfahren zur Herstellung von N-(D)-Ribitylxylidin (I) durch Umsetzen von (D)-Ribose (II) mit 3,4-Dimethyl-anilin (III) oder 3,4-Dimethyl-1-nitro-benzol (IV) in wäßriger oder wäßrig-organischer Lösung oder in der Lösung eines wasserlöslichen organischen Lösungsmittels bei erhöhtem Wasserstoffdruck und in Gegenwart eines Hydrierkatalysators und einer geeigneten Borsäureverbindung, das dadurch gekennzeichnet ist, daß man

a) die Borsäureverbindung in katalytischen Mengen von etwa 6 bis 35 mmol, vorzugsweise 6 bis 20 mmol pro Mol Ribose verwendet,
b) die Umsetzug bei Wasserstoffdrücken von 1 bis 20, vorzugsweise 2 bis 9 bar durchführt und
c) die Hydrierung im Temperaturbereich von 40 bis 80°C, vorzugsweise 50 bis 60°C an Raney-Nickel als Hydrierkatalysator durchführt.

Als geeignete Borsäureverbindungen seien Boroxid, ein Borat oder Borsäure selbst genannt.

Die Erniedrigung des Wasserstoffdruckes bedeutet eine erhebliche Verringerung der Investitionskosten für die Hydrieranlage sowie eine bedeutende Verringerung des verfahrenstechnischen Aufwands.

Durch Zugabe des Borsäurekatalysators wird, wie die Ausführungsbeispiele zeigen, die Ribitylxylidin-Ausbeute um 25 bis 40 % der Theorie (je nach Ausgangsmaterial) gesteigert. Dieser Effekt ist bei jeder Ribosequalität zu beobachten. Die erforderliche Menge an Katalysator liegt bei etwa 6 bis 35 mmol pro mol Ribose. Die genaue Menge hängt von der Qualität der jeweils eingesetzten Ribose ab und kann in einfachen Vorversuchen ermittelt werden.

Die erfindungsgemäße Umsetzung wird im allgemeinen in den für diese Umsetzung geeigneten niederen Alkoholen, insbesondere Methanol, in reiner Form oder auch in Gegenwart von Wasser, durchgeführt. Die Reaktionstemperaturen liegen bei 40 bis 80°C, insbesondere 50 bis 60°C. Der Wasserstoffdruck bei der Umsetzung beträgt im allgemeinen 1 bis 20 bar. Mit Vorteil arbeitet man mit Wasserstoffdrücken zwischen 2 und 9, insbesondere 2 und 5 bar, was in den gängigen Reaktoren möglich ist, da übliche Reaktoren im allgemeinen für Reaktionen unter einem Druck bis zu 10 bar zugelassen sind. Bei den bevorzugten Druckverhältnissen kann man auf die Verwendung von Kompressoren verzichten und den Reaktor aus Wasserstoffflaschen beschicken. Im Prinzip könnte man die Umsetzung sogar unter Normaldruck durchführen, jedoch erhält man hierbei ein geringfügig stärker verunreinigtes Ribamin, so daß man einen leichten $H_2$-Überdruck vorzieht. Die Reaktionszeit beträgt etwa 4 bis 6 Stunden. Nach Abschluß der Umsetzung wird das Raney-Nickel abfiltriert, das Filtrat aufkonzentriert und hieraus durch Zusatz von Wasser Ribitylxylidin in hohen Ausbeuten und Reinheiten ausgefällt.

Geht man bei der erfindungsgemäßen Umsetzung von 3,4-Dimethyl-1-nitrobenzol aus, so empfiehlt es sich dieses vorab im gleichen Lösungsmittel und am gleichen Hydrierkatalysator zum 3,4-Dimethyl-anilin zu hydrieren. Diese Verfahrensvariante wird durch Beispiel 16 veranschaulicht.

Mit Hilfe des erfindungsgemäßen Verfahrens erhält man das als wichtiges Zwischenprodukt für die industrielle Herstellung von Riboflavin begehrte Ribitylxylidin auch bei Verwendung von Raney-Nickel und nur niederen Wasserstoffdrücken auf einfache Weise in sehr guten Ausbeuten.

<u>Beispiele</u>

<u>Allgemeine Arbeitsvorschrift</u>

Jeweils die aus den folgenden Tabellen ersichtliche Menge an 3,4-Dimethyl-anilin (III) wurde in 150 ml Methanol gelöst, die Lösung mit 20 g wasserfeuchtem Raney-Nickel versetzt und das erhaltene Gemisch in einem Autoklaven auf ca. 55°C aufgeheizt. Anschließend wurde innerhalb von 4 Stunden (h) die in den folgenden Tabellen näher definierte methanolische Ribose(II)-Lösung, der ggf. zuvor katalytische Mengen Borsäure zugesetzt worden waren, zugepumpt. Dann wurde mit 60 ml Methanol nachgespült und das Reaktionsgemisch noch 2 h bei 5 bar Wasserstoffdruck und 55°C nachreagieren gelassen. Anschließend wurde das Reaktionsgemisch heiß durch Filtration vom Katalysator abgetrennt und das Filter mit 100 ml Methanol nachgespült. Das Filtrat wurde sodann auf etwa die Hälfte eingeengt und hieraus das N-(D)-Ribitylxylidin (I) durch Zugabe von etwa derselben Menge warmen Wassers ausgefällt. Nach

Abkühlenlassen und 3-stündigem Rühren im Eiswasserbad wurde abgesaugt und die Kristalle mit Wasser und kaltem (O bis 5°C) Methanol gewaschen und unter vermindertem Druck bei 70°C getrocknet.

Beispiele 1 und 2, Vergleichsbeispiel 1

A. Umsetzung mit reiner kristalliner Ribose

Es wurden jeweils 40,0 g (0,267 mol) D-Ribose (II) eingewogen und auf 360 ml mit Methanol aufgefüllt als Riboselösung und 32,9 g (0,272 mol) 3,4-Dimethyl-anilin (III) in 150 ml Methanol eingesetzt.

Tabelle 1

| | | | | Ribitylxylidin (I) | |
|---|---|---|---|---|---|
| Beispiel | III-Einwaage | Borsäure-zusatz | I-Auswaage | I-Ausbeute | Drehwert |
| | [g (mol)] | [mg] | [g] | [% der Theorie] | [$\alpha^{25}_{D}$ (in 2 N HCl] |
| 1 | 32,9 (0,272) | 150 | 61,0 | 89,6 | -37,5° (c=1,3 |
| 2 | 32,9 (0,272) | 150 | 60,7 | 89,2 | -36,5° (c=1,3 |
| Vgl. 1 | 32,9 (0,272) | - | 37,5 | 55,1 | -37,0° (c=1,0 |

Beispiel 3

Man arbeitete wie für die Beispiele 1 und 2 beschrieben, ließ jedoch das Reaktionsgemisch statt 2 h bei 5 bar Wasserstoffdruck, 2 h bei 0,5 bar Wasserstoffdruck nachreagieren. Dabei wurde das N-(D)-Ribitylxylidin in einer Ausbeute von 86 % der Theorie in einer Reinheit von etwa 90 % (Fp. = 133°C; $[\alpha]^{25}_{D}$ = -32,1) gewonnen.

Beispiel 4

Man arbeitete wie für die Beispiele 1 und 2 beschrieben, ließ jedoch das Reaktionsgemisch statt 2 h bei 5 bar Wasserstoffdruck, 2 h bei 1,5 bar Wasserstoffdruck nachreagieren. Dabei wurde das N-(D)-Ribitylxylidin in einer Ausbeute von 85 % der Theorie gewonnen. Fp = 136°C/$[\alpha]^{25}_{D}$ = -34,2.

Beispiele 5 bis 7 und Vergleichsbeispiele 2 bis 4

B. Umsetzung mit einer Ribelösung, die hergestellt wurde durch Ein wiegen der aus der Tabelle 2 ersichtlichen Menge einer Ribose, die u.a. zu 64,6 Gew.% aus Ribose und zu 30,1 Gew.% aus Wasser bestand und Auffüllen mit Methanol auf 360 ml.

Tabelle 2

| Beispiel | II-Einwaage [g | (mol)] | III-Einwaage [g] | Zusatz Borsäure [mg] | Ribitylxylidin (I) I-Auswaage [g] | I-Ausbeute [% der Theorie] | I-Reinheit [HPLC] |
|---|---|---|---|---|---|---|---|
| Vgl. 2 | 60,3 | (0,260) | 33,0 g | - | 34,8 | 52,5 % | 99,4 % |
| Vgl. 3 | 61,54 | (0,265) | 33,6 g | - | 33,6 | 50,7 % | 99,2 % |
| Vgl. 4* | 61,54 | (0,265) | 33,6 g | - | 40,6 | 61,2 %* | 99,4 % |
| 5 | 61,54 | (0,265) | 33,6 g | 150 | 58,8 | 88,7 % | 99,6 % |
| 6 | 61,54 | (0,265) | 33,6 g | 150 | 60,5 | 91,3 % | 99,8 % |
| 7 | 61,54 | (0,265) | 33,6 g | 150 | 59,6 | 89,9 % | 99,5 % |

* bei diesem Versuch wurde 9 h (anstelle von 2 h) bei 55°C und 5 bar $H_2$ nachgerührt.

Beispiele 8 bis 15 und Vergleichsbeispiele 5 bis 11

Ribitylxylidin-Ausbeute in Abhängigkeit vom Borsäurezusatz

C. Umsetzung mit einer Riboselösung, die hergestellt wurde durch Einwiegen von jeweils 75,5 g Rohribose mit einem Ribosegehalt von 47,5 Gew.% (entsprechend 35,86 g (0,239 mol); Gesamtgehalt an reduzierenden Zuckern 67 Gew.%), die gemäß DE-OS 30 04 304 aus Arabinose gewonnen wurde.

| Beispiel | III-Einwaage [g] | Borsäurezusatz [mg | (mmol/mol II)] | Ribitylxylidin Auswaage [g] | Ribitylxylidin Ausbeute [% der Theorie] |
|---|---|---|---|---|---|
| Vgl. 5 | 35,5 (0,293 mol) | - | - | 37,2 | 61,0 % |
| Vgl. 6 | 35,5 | - | - | 37,3 | 61,2 % |
| Vgl. 7 | 35,5 | 10 | ( 0,68) | 40,0 | 65,6 % |
| Vgl. 8 | 35,5 | 20 | ( 1,36) | 41,4 | 67,9 % |
| Vgl. 9 | 35,5 | 50 | ( 3,39) | 48,6 | 79,7 % |
| 8 | 35,5 | 100 | ( 6,78) | 51,3 | 84,2 % |
| 9 | 35,5 | 100 | ( 6,78) | 52,8 | 86,6 % |
| 10 | 35,5 | 100 | ( 6,78) | 52,4 | 86,0 % |
| 11 | 35,5 | 150 | (10,17) | 53,0 | 87,0 % |
| 12 | 35,5 | 150 | (10,17) | 52,6 | 86,3 % |
| 13 | 35,5 | 200 | (13,57) | 53,2 | 87,3 % |
| 14 | 35,5 | 300 | (20,34) | 51,7 | 84,8 % |
| 15 | 35,5 | 400 | (27,14) | 48,0 | 78,8 % |
| Vgl. 10 | 35,5 | 700 | (47,48) | 47,2 | 77,4 % |
| Vgl. 11 | 35,5 | 1200 | (81,0 ) | 46,5 | 76,3 % |

Beispiel 16

Umsetzung ausgehend von 3,4-Dimethyl-1-nitrobenzol

40,32 g (266,6 mmol) 3,4-Dimethyl-1-nitrobenzol und 22 g Raney-Nickel wurden in 200 ml Methanol bei 55°C und 5 bar $H_2$-Druck 2 h hydriert. Gemäß gaschromatographischer Analyse verlief die Hydrierung zum 3,4-Dimethyl-anilin ohne Nebenproduktbildung. Zu diesem Gemisch wurden anschließend nochmals 22 g Raney-Nickel addiert. Bei 5 bar $H_2$-Druck und 55°C wurde dann innerhalb von 4 h 40 g (100 %ig reiner) Ribose und 1,0 g Borsäure, die mit Methanol auf 360 ml aufgefüllt waren, zugepumpt. Dann ließ man noch 2 h bei 5 bar $H_2$-Druck und 55°C nachreagieren. Anschließend wurde der Katalysator bei 60°C mit einer beheizten Drucknutsche abgetrennt. Die Mutterlauge wurde auf 350 g eingeengt, dann 350 g heißes Wasser dazugegeben. Das erhaltene Reaktionsgemisch wurde abkühlen lassen, dann noch 1 h bei Raumtemperatur und 3 h im Eiswasserbad gerührt. Das erhaltene Kristallisat wurde abgesaugt, mit je 50 ml kaltem Wasser und 50 ml kaltem Methanol gewaschen und getrocknet. Man erhielt 58,5 g, entsprechend 86 % der Theorie. $[\alpha]^{25}_D = -37{,}2$.

## Patentansprüche

1. Verfahren zur Herstellung von N-(D)-Ribitylxylidin (I) durch Umsetzen von (D)-Ribose (II) mit 3,4-Dimethyl-anilin (III) oder 3,4-Dimethyl-1-nitro-benzol (IV) in wäßriger oder wäßrig-organischer Lösung oder in der Lösung eines wasserlöslichen organischen Lösungsmittels bei erhöhtem Wasserstoffdruck und in Gegenwart eines Hydrierkatalysators und einer Borsäureverbindung, dadurch gekennzeichnet, daß man
   a) die Borsäureverbindung in katalytischen Mengen von etwa 6 bis 35 mmol, vorzugsweise 6 bis 20 mmol pro Mol Ribose verwendet,
   b) die Umsetzung bei Wasserstoffdrücken von 1 bis 20 bar durchführt und
   c) die Hydrierung im Temperaturbereich von 40 bis 80°C an Raney-Nickel als Hydrierkatalysator durchführt.
2. Verfahren gemäß Anspruch 1, dadurch gekennzeichnet, daß man als Borsäureverbindung Boroxid, ein Borat oder Borsäure selbst verwendet.
3. Verfahren gemäß Anspruch 1, dadurch gekennzeichnet, daß man die Umsetzung bei Wasserstoffdrucken von 2 bis 9 bar durchführt.
4. Verfahren gemäß Anspruch 1, dadurch gekennzeichnet, daß man die Umsetzung bei 50 bis 60°C durchführt.
5. Verfahren gemäß Anspruch 1, dadurch gekennzeichnet, daß man die Umsetzung in Gegenwart von 6 bis 20 mmol der Borsäureverbindung durchführt.

## Claims

1. A process for preparing N-(D)-ribitylxylidine (I) by reacting (D)-ribose (II) with 3,4-dimethylaniline (III) or 3,4-dimethyl-1-nitrobenzene (IV) in aqueous or aqueous/organic solution or in solution in a watersoluble organic solvent at an elevated hydrogen pressure and in the presence of a hydrogenation catalyst and of a boric acid compound, which comprises
   a) using the boric acid compound in a catalytic amount of from about 6 to 35 mmol, preferably from 6 to 20 mmol, per mol of ribose,
   b) carrying out the reaction at a hydrogen pressure of from 1 to 20 bar and
   c) carrying out the hydrogenation at from 40 to 80°C over Raney nickel as hydrogenation catalyst.
2. A process as claimed in claim 1, wherein the boric acid compound used is boron oxide, a borate or boric acid itself.
3. A process as claimed in claim 1, wherein the reaction is carried out at a hydrogen pressure of from 2 to 9 bar.
4. A process as claimed in claim 1, wherein the reaction is carried out at from 50 to 60°C.
5. A process as claimed in claim 1, wherein the reaction is carried out in the presence of from 6 to 20 mmol of the boric acid compound.

## Revendications

1. Procédé de préparation de N-(D)-ribitylxylidine (I) par réaction de (D)-ribose (II) avec la 3,3-diméthylaniline (III) ou le 3,4-diméthyl-1-nitro-benzène (IV) en solution aqueuse ou aqueuse-organique ou dans la solution d'un solvant organique hydrosoluble, sous pression d'hydrogène élevée et en présence d'un catalyseur d'hydrogénation et d'un composé d'acide borique, caractérisé en ce que
   a) on utilise le composé d'acide borique en quantités catalytiques d'environ 6 à 35 mmoles, de préférence 6 à 20 mmoles par mole de ribose,
   b) on mène la réaction à des pressions d'hydrogène de 1 à 20 bar et

c) on mène l'hydrogénation dans une gamme de température de 40 à 80°C sur du nickel de Raney comme catalyseur d'hydrogénation.

2. Procédé selon la revendication 1, caractérisé en ce qu'on utilise comme composé d'acide borique, l'oxyde de bore, un borate ou l'acide borique lui-même.

3. Procédé selon la revendication 1, caractérisé en ce qu'on mène la réaction à des pressions d'hydrogène de 2 à 9 bar.

4. Procédé selon la revendication 1, caractérisé en ce qu'on mène la réaction à 50–60°C.

5. Procédé selon la revendication 1, caractérisé en ce qu'on mène la réaction en présence de 6 à 20 mmoles du composé d'acide borique.